(19) Europäisches Patentamt
European Patent Office
Office européen des brevets

(11) **EP 1 747 757 B1**

(12) **EUROPEAN PATENT SPECIFICATION**

(45) Date of publication and mention
of the grant of the patent:
**14.09.2011   Bulletin 2011/37**

(51) Int Cl.:
*A61B 8/08* (2006.01)

(21) Application number: **06015465.5**

(22) Date of filing: **25.07.2006**

(54) **Ultrasound system for displaying an elastic image**

Ultraschallsystem zur Wiedergabe eines elastischen Bildes

Système à ultrasons pour visualiser une image élastique

(84) Designated Contracting States:
**DE FR IT**

(30) Priority:  **27.07.2005   KR 20050068269**
**28.12.2005   KR 20050131465**

(43) Date of publication of application:
**31.01.2007   Bulletin 2007/05**

(73) Proprietor: **MEDISON CO., LTD.**
**Kangwon-do 250-870 (KR)**

(72) Inventors:
• **Moon, Hae Yean**
**Discusser & Medison Building**
**Seoul 135-280 (KR)**

• **Kim, Cheol An**
**Discusser & Medison Building**
**Seoul 135-280 (KR)**
• **Yoon, Ra Young**
**Discusser & Medison Building**
**Seoul 135-280 (KR)**

(74) Representative: **Schmid, Wolfgang**
**Lorenz & Kollegen**
**Patentanwälte Partnerschaftsgesellschaft**
**Alte Ulmer Strasse 2**
**89522 Heidenheim (DE)**

(56) References cited:
**EP-A- 1 554 982        JP-A- 2005 066 041**
**US-A- 4 926 871        US-A- 5 497 249**
**US-A1- 2005 119 568**

**Description**

The present invention generally relates to an ultrasound system, and more particularly to an ultrasound system for forming and displaying an elastic image that provides tissue information of a target object by measuring a modulus of tissue elasticity.

An ultrasound diagnostic system has become an important and popular diagnostic tool since it has a wide range of applications. Specifically, due to its non-invasive and non-destructive nature, the ultrasound diagnostic system has been extensively used in the medical profession. Modern high-performance ultrasound diagnostic systems and techniques are commonly used to produce two or three-dimensional diagnostic images of internal features of an object (e.g., human organs).

The ultrasound diagnostic system generally uses a wide bandwidth transducer to transmit and receive ultrasound signals. The ultrasound diagnostic system forms images of human internal tissues by electrically exciting an acoustic transducer element or an array of acoustic transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which are amplified and processed to produce ultrasound data for an image of the tissues. The ultrasound diagnostic system is very important in the medical field since it provides physicians with real-time and high-resolution images of human internal features without the need for invasive observation techniques such as surgery.

The ultrasound image is usually displayed in a Brightness-mode (B-mode) using reflectivity caused by an impedance difference between the tissues of the target object. However, if the reflectivity of the target object is hardly different from those of the neighboring tissues such as tumor, cancer or the like, then it is not easy to recognize the target object in the B-mode image. Further, an ultrasound elastic imaging technology has been developed to display an image of the target object by using mechanical characteristics of the target object. Such technology is very helpful for diagnosing lesions such as cancers.

The conventional elastic imaging technology displays the image of the target object by mapping relative strains of the tissues, which occur by applying stress to the target object, using pseudo colors. Such technology using pseudo colors is advantageous since it can easily display information relating to tissue hardness. However, there is a problem with the above technology in that the tissue hardness cannot be quantitatively displayed.

Also, it is difficult to clearly display the boundaries between the lesion and neighboring tissues with the conventional elastic imaging technology. Further, such technology is highly inconvenient since various operations must be carried out in order to measure information relating to radius and circumference of a tissue in an obtained image.

JP 2005 066041 A discloses an ultrasound system according to the preamble of claim 1.

In EP-A-1 554 982, there is described an ultrasound diagnostic apparatus including means for generating a thermographic image from a reflected echo signal received by the probe.

US 2005/119568 A1 describes an imaging system comprising a device to excite mechanical waves in elastic tissue, a device for measuring the resulting tissue motion at a plurality of locations interior to the tissue at a number of time instances, a computing device to calculate the mechanical properties of tissue from the measurements, and a display to show the properties according to their location.

An apparatus and a method for measuring the volume of urine in a human bladder is known from US-A-4 926 871. The apparatus includes a scanhead and transceiver for transmitting a plurality of ultrasound signals into the bladder and for receiving the returning ultrasound signals. The area of the bladder is is calculated by summing successive trapezoid formed by successive A-line distances representing distances between the back and from borders of the bladder, which are measured while the transducer moves within the scanhead assembly.

US-A-5 497 249 discloses an apparatus which performs filter processing on original image data, wherein an image data statistical processing unit is used to extract and statistically processing the characteristics of picture element distribution in the picture expressed by the original image data.

The present invention provides an ultrasound system for forming an ultrasound image by measuring a modulus of elasticity of tissues and then forming and displaying an elastic image indicating the measured information of a target object.

According to the present invention, there is provided an ultrasound system according to claim 1.

The above and other objects and features of the present invention will become apparent from the following descriptions of preferred embodiments given in conjunction with the accompanying drawings, in which:

Fig. 1 is a block diagram showing an ultrasound system constructed in accordance with the present invention;
Fig. 2 is a schematic diagram showing a spring model for explaining a modulus of elasticity;
Fig. 3 is a schematic diagram showing a structure of a probe constructed in accordance with the present invention;
Fig. 4 is a diagram showing an example of a selection window;

Fig. 5A is a photograph showing an elastic image before an image enhancing process is performed;
Fig. 5B is a photograph showing an elastic image in which an image enhancing process is performed; and
Fig. 6 is a photograph showing an elastic image performed in an automatic measure mode in accordance with the present invention.

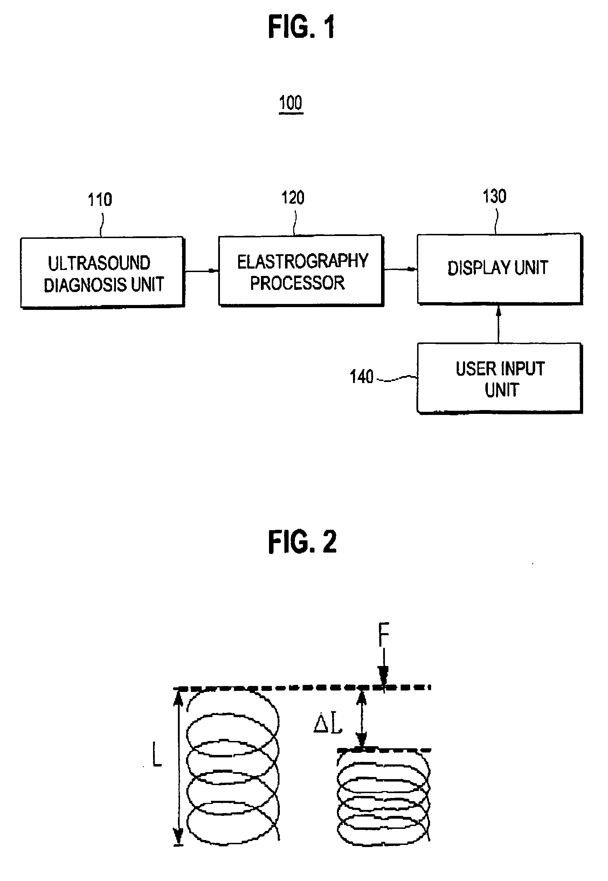

Fig. 1 is a block diagram showing an ultrasound system constructed in accordance with the preferred embodiment of the present invention. As shown in Fig. 1, the ultrasound system 100 includes an ultrasound diagnosis unit 110, an elastography processor 120, a display unit 130 and a user input unit 140. The ultrasound diagnosis unit 110 includes a probe having a stress applying unit around a scan surface thereof and a stress measuring sensor positioned on the stress applying unit. The ultrasound diagnosis unit 100 provides ultrasound scanning information of the target object and strength information of the stress applied to the target object. The target object may be a reflector included in a subject. Further, the probe may be a 3-dimensional probe.

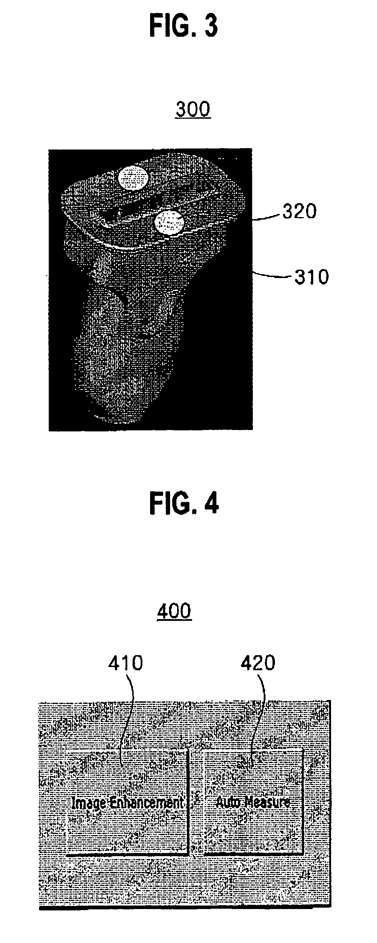

The information relating to the stress applied to the tissues should be obtained in order to accurately form an elastic image and to quantitatively measure the modulus of elasticity of the tissues. In order to deeply and uniformly apply the stress to the target object along a vertical direction, a probe, the scan surface of which is provided with a stress applying unit 310, is employed (shown in Fig. 3). The stress applying unit 310 has an opening exposing the scan surface of the probe at a center thereof. The contact surface of the stress applying unit 310 should be greater than the scan surface of the probe so that the stress can be more deeply and uniformly applied to the tissues within the human body. A stress measuring sensor 320 may be attached to the contact surface of the stress applying unit 310. The strength information of the stress measured by the stress measuring sensor 320 is transferred to the elastic image processor 120. The stress measuring sensor 320 may be a cable-type or wireless-type sensor.

The elastic image processor 120 forms an elastic image based on the ultrasound scanning information and the strength information of the stress. If the stress measuring sensor 320 is a cable-type sensor, then the strength information of the stress is directly transmitted from the stress measuring sensor 320 to the elastic image processor 120. However, if the stress measuring sensor 320 is a wireless-type sensor, then the ultrasound system 100 may further include a wireless signal receiving unit and a wireless signal processing unit (not shown). The strength information of the stress is transmitted to the elastic image processor 120 through the wireless signal processing unit.

The modulus of elasticity may be obtained by measuring the strain obtained by comparing the ultrasound signals reflected from the target object before and after the stress is applied. The modulus of elasticity may be defined by using the 1-dimensional spring model illustrated in Fig. 2. A force F required for compressing the spring by a predetermined depth is proportional to the modulus of elasticity. That is, if the stress applied to a unit area is σ and the strain is s, then the modulus of elasticity (E) is defined as the following equation:

$$E = \frac{\sigma}{\varepsilon} \quad (\sigma = F/A, \varepsilon = \Delta L/L) \qquad\qquad (1)$$

Wherein "A" represents an area to which the stress is applied, "L" represents a length of the spring not applying the stress, and "ΔL" represents a length variation of the spring according to the stress applied. The modulus of elasticity is referred to as the Young's modulus. The modulus of elasticity of the target object is measured by calculating the ratio of a length not subjected the stress to a length subjected to the stress. However, since the degree of the strain of the tissues in the human body cannot be directly measured, the strain is usually measured with the assumption that the stress is uniformly distributed in the human body. The elastic image is formed based on the measured strain.

The user input unit 140 receives an input from a user for selecting an image enhancement mode or an automatic measure mode for the elastic image. A selection window can be displayed on a screen of the display unit 130 displaying the current elastic image for mode selection (shown in Fig. 4). The selection widow provides a first button 410 for selecting an image enhancement mode and a second button 420 for selecting an automatic measure mode. If the image enhancement mode is selected by the user, then the image processor 120 performs image processing to clearly separate the boundaries between the lesions and neighboring tissues.

Hereinafter, an image enhancing method, which is performed in the image processor 120, will be described. If the selection information for selecting the image enhancement mode is inputted through the user input unit 140, then a mask of M×N size is applied to an elastic image currently displayed on a screen, wherein M and N are positive integers. Then, a threshold value is set based on the color values of entire pixels in the elastic image. Subsequently, a sum of the differences of the color values between neighboring pixels, which exist in the M×N mask, is calculated and the calculated sum is compared with the threshold value. If the sum is smaller than the threshold value, then the elastic image is smoothened by using a convolution mask, which is generally used to blur the image. According to the above process, normal tissues are displayed in a relatively smooth image in comparison to the lesion tissues.

If the sum is greater than the threshold value, a sharpening process is carried out for the elastic image. The sharpening process is carried out by applying a high-pass filter to the M×N mask. Since color differences between the lesion tissues are greater than those between the normal tissues, the boundaries between the lesion tissues and the normal tissues can be further sharpened through the sharpening process.

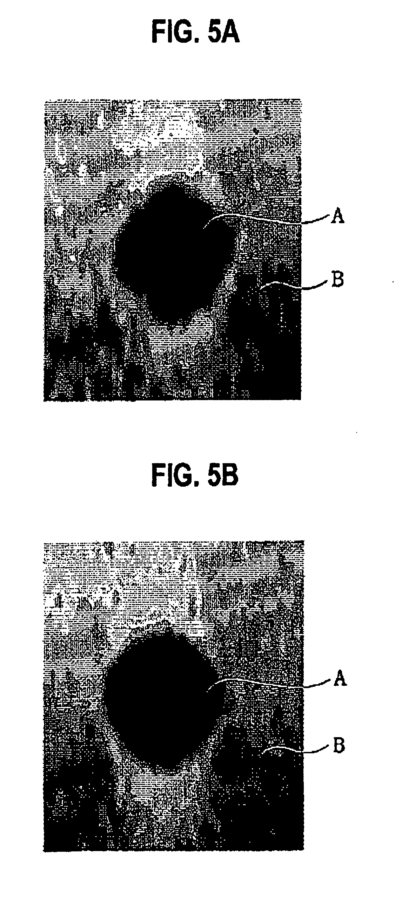

Fig. 5A is a photograph showing an elastic image before the image enhancement process is performed. Fig. 5B is a photograph showing an elastic image after performing the image enhancement process of the present invention. As shown in Figs. 5A and 5B, an enhanced elastic image can be obtained through the image enhancement process of the present invention. Symbols A and B in Figs. 5A and 5B represent the lesion and normal tissues (adjacent to the lesion), respectively.

Hereinafter, the operation of the automatic measure mode will be described. If the automatic measure mode is selected by the user through the selection window shown in Fig. 4, then a user interface such as a mouse pointer is activated on the elastic image. A specific lesion is selected by the user through the user interface and then the user interface is inactivated. After the lesion is selected, an automatic measure function used in a conventional ultrasound system is executed. This is so that the boundaries of the selected lesion are extracted and the diameter of the lesion is indicated based on the boundary information of extracted lesion. Different color information between the normal tissues and lesion tissues is used to trace the normal tissues and the lesion tissues. Trace coordinates (horizontal and vertical) of the boundaries between the normal tissues and the lesion tissues are stored. The difference between the minimum coordinate value and the maximum coordinate value is used to calculate the diameter of the lesion.

Further, an area of the lesion is calculated by using the stored trace coordinates and the coordinates of a mouse point selecting the lesion through the user interface. The areas of triangles drawn with the coordinates of the mouse point and the two traced coordinates are calculated. Then, the calculated areas are summed to calculate an area of the lesion. In order to calculate the area of the triangle, one of the trace coordinates is selected as first trace coordinates and a threshold is set to select the second trace coordinates around the first trace coordinates. If the area of the lesion is calculated by using all trace coordinates, then errors occurring in the area calculation of the triangles can be accumulated. Therefore, the threshold is set to minimize the errors.

The second trace coordinates are selected in a predetermined direction by using the threshold. The second trace coordinates correspond to coordinates, which render the distance difference between the first trace coordinates and the neighboring trace coordinates to be most approximate to the threshold. A first area of a first triangle drawn by the first trace coordinates, the second trace coordinates and the coordinates of the mouse point is calculated. Subsequently, third trace coordinates are selected by using the threshold. Further, a second area of a second triangle drawn by the second trace coordinates, the third trace coordinates and the coordinates of the mouse point is calculated. A fourth, fifth ... $n^{th}$ trace coordinates are selected and the areas of the triangles are calculated as in the above process. The first trace coordinates are selected to calculate an area of a last triangle regardless of the threshold. That is, the last triangle is drawn by the nth trace coordinates, the first trace coordinates and the coordinates of the mouse point. The calculated diameter and the area of the lesion are indicated on the elastic image.

Also, if the user selects the automatic measure mode, then a volume of lesion can be measured in a 3-dimensional space by using 3-dimensional data obtained by using the 3-dimensional probe. The boundaries between the lesion and the normal tissues can be detected in the same manner as mentioned above. If the lesion is a sphere, then the volume of the lesion can be calculated by the equation of $\frac{4}{3}\pi r^3$ . However, since the lesion cannot be considered as a sphere, the volume of the lesion is calculated by measuring the diameters in axial, lateral and elevational directions. If the diameters in the axial, lateral and elevational directions are x, y and z, respectively, then the volume of the lesion can be approximately calculated by using the equation of $\frac{4}{3}\pi xyz$ . The calculated volume is displayed on the elastic image display unit.

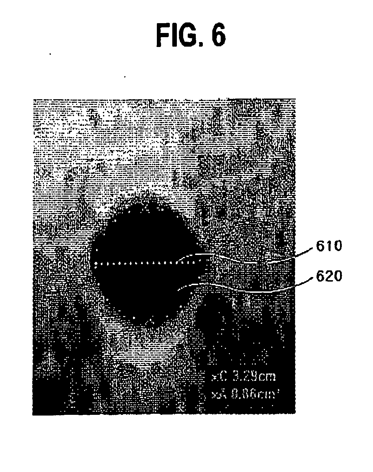

Fig. 6 is a photograph showing an elastic image performed in the automatic measure mode of the present invention. If the user selects a lesion on the elastic image displayed on the display unit and the automatic measure mode in the selection window shown in Fig. 3, then a diameter 610 and an area 620 of the selected lesion are automatically measured and displayed on the display unit (shown in Fig. 6).

As mentioned above, since the modulus of the elasticity is quantitatively calculated and displayed in a numerical value on the elastic image display unit, the hardness of the lesion can be perceived intuitively. Therefore, the lesion can be easily diagnosed.

Also, the present invention provides the enhancement mode and the automatic measure mode of the elastic image so that a desirable elastic image can be provided. Further, since the diameter and the area of the lesion can be automatically measured and the volume of the lesion in the 3-dimensional space can be measured by using the volume data, the characteristics of the lesion can be rapidly and conveniently diagnosed.

While the present invention has been described and illustrated with respect to a preferred embodiment of the invention, it will be apparent to those skilled in the art that variations and modifications are possible without deviating from the broad principles and teachings of the present invention, which should be limited solely by the scope of the claims appended hereto.

## Claims

1. An ultrasound system (100), comprising: an ultrasound diagnosis unit (110) for providing ultrasound scanning information of a target object and stress information applied to the target object, the ultrasound diagnosis unit (110) including a probe having a stress applying unit and a stress measuring sensor around a scan plane thereof; an elastic image processor (120) for forming an elastic image based on the ultrasound scanning information and the stress information; and a display unit (130) for displaying the elastic image, wherein the elastic image processor is adapted to calculate a strain of the target object based on ultrasound receiving signals obtained before and after applying the stress, wherein the elastic image processor is adapted to calculate a modulus of elasticity of the target object based on the stress information and the strain, and wherein the elastic image processor is adapted to form the elastic image based on the ultrasound scanning information and the modulus of elasticity of the target object, **characterized in that** the ultrasound system further comprises a user input unit (140) for receiving an input from a user to select one of an image enhancement mode and an automatic measure mode, wherein, if the image enhancement mode is selected through the user input unit (140), the elastic image processor (120) is adapted to set a threshold value based on color values of entire pixels in the elastic image, apply a mask to the elastic image, smoothen the elastic image by using a convolution mask when a sum of differences of the color values existing in the mask is smaller than the threshold value, and sharpen the elastic image by using high-pass filter when the sum is greater than the threshold value, and if the automatic measure mode is selected through the user input unit (140), a specific lesion is selected through a user interface activated on the elastic image and the elastic image processor (120) is adapted to trace boundaries of the selected lesion, wherein the elastic image processor (120) is adapted to receive the coordinates of a mouse point designated through the user interface, select first trace coordinates from a plurality of trace coordinates of the boundaries, and for i = 1...n-1, **i** being an integer:

   select $(i+1)^{th}$ trace coordinates around the $i^{th}$ trace coordinates in a predetermined direction and so that the distance difference between the $i^{th}$ trace coordinates and the $(i+1)^{th}$ trace coordinates is most approximate to a threshold and calculate the area of a triangle drawn by the $i^{th}$ and $(i+1)^{th}$ trace coordinates and the coordinates of the mouse point designated through the user interface,

   the elastic image processor (120) further adapted to calculate the area of the last triangle drawn by the $n^{th}$ trace coordinates and the first trace coordinates and the coordinates of the mouse point designated through the user interface, the elastic image processor (120) being further adapted to sum the calculated areas of the triangles to measure an area of the selected lesion, wherein the display unit (130) is adapted to further display the measured result together with the elastic image.

## Patentansprüche

1. Ultraschallsystem (100), welches Folgendes aufweist: eine Ultraschalldiagnoseeinheit (110) zum Erzeugen einer Ultraschall-Scaninformation eines Zielobjekts und von auf das Zielobjekt aufgebrachter Belastungsinformationen, wobei die Ultraschalldiagnoseeinheit (110) eine Messsonde aufweist, welche eine Belastungsaufbringungseinheit und einen Belastungsmesssensor um eine Scanebene derselben aufweist; einen elastischen Bildprozessor (120) zum Bilden eine elastischen Bilds basierend auf der Ultraschall-Scaninformation und der Belastungsinformation; und eine Anzeigeeinheit (130) zum Anzeigen des elastischen Bilds, wobei der elastische Bildprozessor dafür vorgesehen ist, eine Dehnung des Zielobjekts basierend auf Ultraschallempfangssignalen zu berechnen, welche vor und nach der Aufbringung der Belastung erhalten wurden, wobei der elastische Bildprozessor dafür vorgesehen ist, einen Elastizitätsmodul des Zielobjekts basierend auf der Belastungsinformation und der Dehnung zu berechnen, und wobei der elastische Bildprozessor dafür vorgesehen ist, das elastische Bild basierend auf der Ultraschall-Scaninformation und dem Elastizitätsmodul des Zielobjekts zu bilden, **dadurch gekennzeichnet, dass** das Ultraschallsystem des Weiteren eine Benutzereingabeeinheit (140) zum Empfangen einer Eingabe von einem Benutzer aufweist, um einen Bildverbesserungsmodus oder einen automatischen Messmodus auszuwählen, wobei, wenn der Bildverbesserungsmodus durch die Benutzereingabeeinheit (140) ausgewählt wird, der elastische Bildprozessor (120) dafür vorgesehen ist, einen Grenzwert basierend auf Farbwerten der gesamten Pixel in dem elastischen Bild festzulegen, eine Maske an dem elastischen Bild anzuwenden, das elastische Bild unter Verwendung einer Konvolutionsmaske zu glätten, wenn eine Summe von Differenzen der in der Maske existierenden Bildwerte kleiner ist als der Grenzwert, und das elastische Bild unter Verwendung eines Hochpassfilters schärfer zu machen, wenn die Summe größer als der Grenzwert ist, und dass, wenn der automatische Messmodus durch die Benutzereingabeeinheit (140) ausgewählt wird, eine spezifische Wunde durch eine auf dem elastischen Bild aktivierte Benutzerschnittstelle ausgewählt wird, und dass der elastische Bildprozessor (120) dafür vorgesehen ist, Begrenzungen der ausgewählten Wunde aufzuzeichnen, wobei der elastische Bildprozessor (120) dafür vorgesehen ist, die Koordinaten eines durch die Benutzerschnittstellen ausgewählten Mauspunkts zu empfangen, erste Aufzeichnungskoordinaten von einer Vielzahl von Aufzeichnungskoordinaten der Begrenzungen auszuwählen, und für i = 1...n-1, wobei i eine ganze Zahl ist:

Auswählen der (i+1)-ten Aufzeichnungskoordinaten um die i-ten Aufzeichnungskoordinaten in einer vorbestimmten Richtung und derart, dass die Abstandsdifferenz zwischen den i-ten Aufzeichnungskoordinaten und den (i+1)-ten Aufzeichnungskoordinaten möglichst nah an einem Grenzwert sind, und Berechnen der Fläche eines Dreiecks, welches von den i-ten und (i+1)-ten Aufzeichnungskoordinaten und den Koordinaten des durch die Benutzerschnittstelle ausgewählten Mauspunkts gebildet wird,

wobei der elastische Bildprozessor (120) des Weiteren dafür vorgesehen ist, die Fläche des letzten Dreiecks zu berechnen, welches durch die n-ten Aufzeichnungskoordinaten und die ersten Aufzeichnungskoordinaten und die Koordinaten des durch die Benutzerschnittstelle ausgewählten Mauspunkts gebildet ist, wobei der elastische Bildprozessor (120) des Weiteren dafür vorgesehen ist, die berechneten Flächen der Dreiecke zu addieren, um eine Fläche der ausgewählten Wunde zu messen,
wobei die Anzeigeeinheit (130) dafür vorgesehen ist, das gemessene Ergebnis zusammen mit dem elastischen Bild anzuzeigen.

## Revendications

1. Système ultrasonore (100), comprenant: une unité de diagnostic ultrasonore (110) pour fournir une information de balayage ultrasonore d'un objet cible et une information de contrainte appliquée à l'objet cible, l'unité de diagnostic ultrasonore (110) comprenant une sonde ayant une unité d'application de contrainte et un capteur de mesure de déformation autour de son plan de balayage ; un processeur d'image élastique (120) pour former une image élastique sur la base de l'information de balayage ultrasonore et de l'information de contrainte ; et une unité d'affichage (130) pour afficher l'image élastique, dans lequel le processeur d'image élastique est agencé pour calculer une déformation de l'objet cible sur la base de signaux de réception ultrasonores obtenus avant et après l'application de la contrainte, dans lequel le processeur d'image élastique est agencé pour calculer un module d'élasticité de l'objet cible sur la base de l'information de contrainte et de la déformation, et dans lequel le processeur d'image élastique est agencé pour former l'image élastique sur la base de l'information de balayage ultrasonore et du module d'élasticité de l'objet cible, **caractérisé en ce que** le système ultrasonore comprend en outre une unité d'entrée utilisateur (140) pour recevoir une entrée d'un utilisateur pour sélectionner l'un ou l'autre d'un mode d'amélioration de l'image et d'un mode de mesure automatique, et dans lequel, si le mode d'amélioration de l'image est sélectionné au moyen de l'unité entrée utilisateur (140), le processeur d'image élastique (120) est agencé pour définir une valeur seuil sur la base des valeurs de couleur de pixels entiers de l'image élastique, appliquer un masque à l'image élastique, adoucir l'image élastique en utilisant un masque de convolution lorsqu'une somme de différences des valeurs de couleurs existant dans le masque est inférieure à la valeur seuil, et accentuer la netteté de l'image élastique en utilisant un filtre passe-haut lorsque la somme est supérieure à la valeur seuil et, si le mode mesure automatique est sélectionné au moyen de l'unité entrée utilisateur (140), une lésion spécifique est sélectionnée au moyen d'une interface utilisateur activée sur l'image élastique et le processeur d'image élastique (120) est agencé pour tracer des frontières de la lésion sélectionnée, dans lequel le processeur d'image élastique (120) est agencé pour recevoir les coordonnées d'un point de souris désigné au moyen de l'interface utilisateur, sélectionner des premières coordonnées de trace à partir d'une pluralité de coordonnées de trace des frontières, et pour i = 1...n-1, i étant un nombre entier :

sélectionner des $(i+1)^{\text{èmes}}$ coordonnées de trace autour des $i^{\text{èmes}}$ coordonnées de trace dans une direction prédéterminée et de façon que la différence de distance entre les $i^{\text{èmes}}$ coordonnées de trace et les $(i+1)^{\text{èmes}}$ coordonnées de trace soit rapprochée au maximum d'un seuil, et calculer la surface d'un triangle dessiné par

EP 1 747 757 B1

les $i^{emes}$, les $(i+1)^{èmes}$ coordonnées de trace et les coordonnées du point de souris désigné au moyen de l'interface utilisateur,

le processeur d'image élastique (120) étant en outre agencé pour calculer la surface du dernier triangle dessiné par les $n^{ièmes}$ coordonnées de trace, les premières coordonnées de trace et les coordonnées du point de souris désigné au moyen de l'interface utilisateur, le processeur d'image élastique (120) étant en outre agencé pour faire la somme des surfaces calculées des triangles pour mesurer une surface de la lésion sélectionnée, et
dans lequel l'unité d'affichage (130) est agencée pour afficher en outre le résultat mesuré en même temps que l'image élastique.

7

# FIG. 1

<u>100</u>

```
┌──────────────┐     ┌──────────────┐     ┌──────────────┐
│  110         │     │  120         │     │  130         │
│  ULTRASOUND  │ ──> │ ELASTROGRAPHY│ ──> │ DISPLAY UNIT │
│  DIAGNOSIS   │     │ PROCESSOR    │     │              │
│  UNIT        │     │              │     │              │
└──────────────┘     └──────────────┘     └──────────────┘
                                                  ▲
                                          ┌──────────────┐
                                    140 ─ │ USER INPUT   │
                                          │ UNIT         │
                                          └──────────────┘
```

# FIG. 2

# FIG. 3

<u>300</u>

—320

—310

# FIG. 4

<u>400</u>

410        420

Image Enhancement     Auto Measure

## FIG. 5A

## FIG. 5B

## FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005066041 A
- EP 1554982 A
- US 2005119568 A1
- US 4926871 A
- US 5497249 A